(19) Europäisches Patentamt
European Patent Office
Office européen des brevets

(11) Veröffentlichungsnummer: **0 060 999**
**B1**

# (12) EUROPÄISCHE PATENTSCHRIFT

(45) Veröffentlichungstag der Patentschrift: **21.08.85**

(51) Int. Cl.⁴: **C 07 H 15/04**, A 61 K 39/00, G 01 N 33/48 // C07H3/04

(21) Anmeldenummer: **82101266.3**

(22) Anmeldetag: **19.02.82**

(54) **Galactopyranoside Verbindungen, Verfahren zu ihrer Herstellung und ihre Verwendung als Antigene oder Immunadsorbentien.**

(30) Priorität: **24.02.81 DE 3106815**

(43) Veröffentlichungstag der Anmeldung: **29.09.82 Patentblatt 82/39**

(45) Bekanntmachung des Hinweises auf die Patenterteilung: **21.08.85 Patentblatt 85/34**

(84) Benannte Vertragsstaaten: **AT BE CH DE FR GB IT LI LU NL SE**

(56) Entgegenhaltungen:
**DE - A - 2 630 596**

**CHEMICAL SOCIETY REVIEWS 1978, 7(4), Seiten 423-452 R.U. LEMIEUX: "Human blood groups and carbohydrate chemistry"**
**TETRAHEDRON LETTERS, Band 22, Nr. 15, 1981, Seiten 1387-1390, Pergamon Press Ltd., G.B. J.C. JACQUINET et al.: "Synthese von Oligosaccharid-Determinanten des T-Antigens mit Amid-Spacer zur Darstellung synthetischer Antigene"**
**Chem. Ber. Band 114 (1981) Seiten 306-327**
**Chem. Soc. Rev. Band 13 (1984) Seiten 20-22**

(73) Patentinhaber: **BEHRINGWERKE AKTIENGESELLSCHAFT, Postfach 1140, D-3550 Marburg/Lahn (DE)**

(72) Erfinder: **Kolar, Cenek, Dr., Lindenweg 14, D-3550 Marburg/Lahn (DE)**
Erfinder: **Paulsen, Hans, Prof. Dr., Hinsbeker Berg 11, D-2000 Hamburg 65 (DE)**
Erfinder: **Jacquinet, Jean Claude, Dr., Allée André Gide 1, F-45100 Orléans-La Source (FR)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**EP 0 060 999 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Verbindungen der allgemeinen Formel I

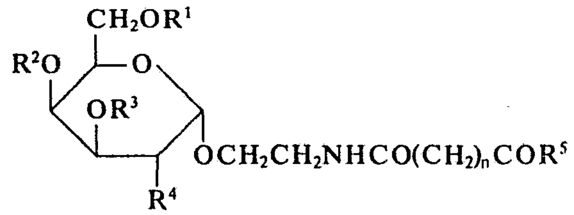

(I)

worin

| | |
|---|---|
| $R^1$ | ein Wasserstoffatom oder eine Acyl- oder Benzyl-Schutzgruppe, |
| $R^1$ und $R^2$ | zusammen eine Alkyliden- oder Benzyliden-Schutzgruppe, |
| $R^2$ und $R^3$ | Wasserstoffatome oder Acyl- oder Benzyl-Schutzgruppen oder einen $\beta$-D-Galactopyranosyl-Rest oder dessen 2,3,4,6-Tetra-O-acetyliertes Derivat, |
| $R^4$ | eine Azido-, Amino- oder Acetamido-Gruppe, |
| $R^5$ | $-$O-Alkyl, $-$O-Aryl, O-Benzyl, $-NHNH_2$, $N_3$ oder $-$OH und |
| n | eine ganze Zahl von 1 bis 10 bedeuten, |

ein Verfahren zu ihrer Herstellung und ihrer Verwendung, gebunden an einen Träger, als künstliche Antigene oder Immunoadsorbentien.

Durch Einwirken von Neuraminidase auf rote Blutkörperchen kann eine antigene Struktur freigelegt werden, die T-aktive antigene Determinante genannt wird.

Dabei handelt es sich um das Disaccharid-Derivat $\beta$-D-Gal(1 $\rightarrow$ 3)-D-GalNAc, das alpha-glykosidisch an einen Serin- oder Threoninrest eines Glykoproteins gebunden ist. Es kommt in vielen Glykoproteinen und -lipiden vor.

Diese T-Determinante wird auch in Tumor-assoziierten Antigenen gefunden.

Für die Herstellung vielfältig verwendbarer künstlicher Antigene und Immunadsorbentien mit Strukturmerkmalen der T-Determinante werden chemische Verbindungen mit diesen Strukturmerkmalen gebraucht, die mit geeigneten Trägermolekülen umgesetzt werden können.

Aus der deutschen Offenlegungsschrift 26 30 596 ist bekannt, daß Kohlenhydrate, deren Hapteneigenschaft bekannt war, durch Bindung an einen Träger über einen Spacer zu künstlichen Antigenen werden, wobei der Spacer ein Rest der Formel $-$RCO$-$R″ ist, worin R einen aliphatischen Kohlenwasserstoffrest mit 3 bis 17 Kohlenstoffatomen und R″ H, OH, $NH_2$, $NHNH_2$, $N_3$, O-Alkyl oder O-Aryl bedeuten.

In Carbohydrate Research 79, C1$-$C7 (1980) ist die Synthese der L-Serin- und L-Threonin-alpha-Glycoside von N-Acetyl-D-Galactosamid beschrieben.

Die vorliegende Erfindung hatte sich zur Aufgabe gestellt, künstliche T-aktive Antigene herzustellen, in denen außer dem Kohlenhydrat-Teil auch die erste glykosidisch gebundene Aminosäure (Serin oder Threonin) durch ein Strukturelement vertreten ist.

Gelöst wird diese Aufgabe durch die Herstellung von Verbindungen der angegebenen allgemeinen Formel I.

Bevorzugt werden im Rahmen der Erfindung Verbindungen der Formel IV

$\alpha$-D-GalNAc-$OCH_2CH_2NHCO(CH_2)_nCOOCH_3$ (IV)

der Formel V

$\beta$-D-Gal(1$-$3)-$\alpha$-D-GalNAc-$OCH_2CH_2NHCO(CH_2)_nCOOCH_3$ (V)

der Formel VI

$\beta$-D-Gal(1$-$4)-$\alpha$-D-GalNAc-$OCH_2CH_2NHCO(CH_2)_nCOOCH_3$ (VI)

und der Formel

$\beta$-D-Gal(1$-$4)-$\alpha$-D-GalNAc-$OCH_2CH_2NHCO(CH_2)_nCOOCH_3$ (VII)
3
1
$\beta$-D-Gal

wobei n = 4 oder 7 ist.

Das erfindungsgemäße Verfahren zur Herstellung der oben beschriebenen neuen Verbindungen ist dadurch gekennzeichnet, daß man

a) eine Verbindung der allgemeinen Formel VIII

$HOOC(CH_2)_nCOR^5$ (VIII)

in der $R^5$ O-Alkyl, O-Aryl oder O-Benzyl und n = 1 bis 10, vorzugsweise 4 bis 7 bedeuten, mit 2-Amino-ethanol $H_2N—CH_2—CH_2OH$ in an sich bekannter Weise zu dem Reaktionsprodukt der allgemeinen Formel III

$HOCH_2CH_2NHCO(CH_2)_nCOR^5$ (III)

umsetzt

b) das Produkt von Stufe a) in an sich bekannter Weise mit einer Verbindung der allgemeinen Formel II

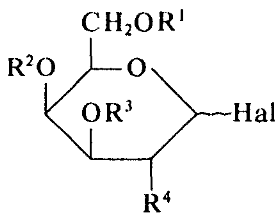

(II)

in der $R^1$, $R^2$, $R^3$ Acylgruppen, bevorzugt Acetyl- oder Benzoyl-Gruppen, $R^4 = —N_3$ und Hal = Cl oder Br bedeuten, bevorzugt zu einem alpha-Glycosid der Formel I umsetzt,

c) in dem Produkt von Stufe b) in an sich bekannter Weise die 2-Azido-Gruppe umsetzt, N-acetyliert und die O-Acetyl-Gruppen alkalisch abspaltet, womit die Verbindung der Formel IV entsteht,

d) das Produkt der Formel IV von Stufe c) in an sich bekannter Weise zu einer 4,6-O-Alkyliden- oder -Benzyliden-Verbindung der allgemeinen Formel IX

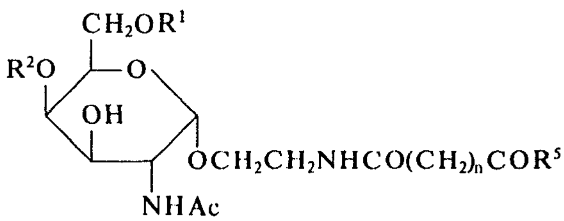

(IX)

worin $R^1$ und $R^2$ zusammen die Benzyliden- oder eine Alkyliden-Gruppe, vorzugsweise die Isopropyliden-Gruppe bedeuten, umsetzt, und entweder

e) das Produkt der Formel IX von Stufe d) in an sich bekannter Weise mit einer Verbindung der allgemeinen Formel X

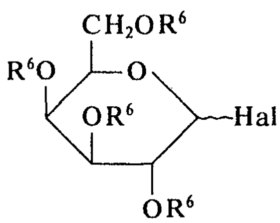

(X)

worin $R^6$ eine Acylgruppe, bevorzugt Acetyl oder Benzoyl, und Hal Br oder Cl ist, zu einem $\beta$-verknüpften Disaccharid-Derivat der allgemeinen Formel XI

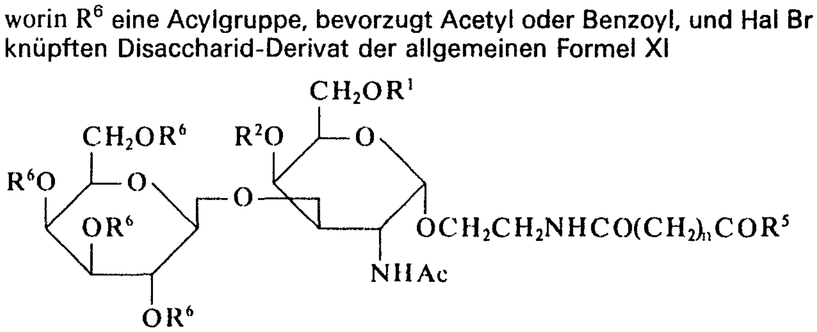

(XI)

umsetzt,

f) das Produkt der Formel XI von Stufe e) in an sich bekannter Weise

1. hydrolytisch mit Säuren oder hydrogenolytisch mit Wasserstoff in Gegenwart von Katalysator unter Abspaltung der Alkyliden- bzw. Benzyliden-Schutzgruppen und
2. alkalisch behandelt, wobei eine Verbindung der Formel V entsteht,

g) das Produkt der Formel XI von Stufe e) in an sich bekannter Weise wie unter f) 1. behandelt und die freie 6-OH-Gruppe mit einer Acyl-Gruppe, bevorzugt einer Acetyl- oder Benzoyl-Gruppe, schützt, wobei die Verbindung der allgemeinen Formel XII

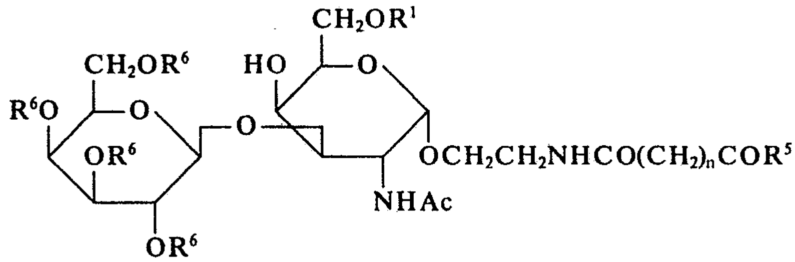

(XII)

worin $R^1$ und $R^6$ Acyl, bevorzugt Acetyl oder Benzoyl, bedeuten, ensteht,

h) das Produkt der Formel XII von Stufe g) in an sich bekannter Weise wie bei Stufe e) mit dem Halogenid der Formel X zu einem Trisaccharid-Derivat umsetzt und nach alkalischer Behandlung die Trisaccharid-Brückenbildungsarm-Verbindung der Formel VII gewinnt, oder

i) in dem Produkt der Formel IX aus der Stufe d) in an sich bekannter Weise die 3-OH-Gruppe der N-Acetyl-D-galactosamin-Einheit mit einer Schutzgruppe, bevorzugt einer Benzyl-, Acetyl- oder Benzoyl-Gruppe versieht, anschließend die 4,6,-O-Benzyliden-Gruppe hydrolytisch abspaltet und schließlich die 6-OH-Gruppe selektiv mit einer Schutzgruppe, bevorzugt einer Acetyl-, oder Benzoyl-Gruppe schützt, wodurch man eine Verbindung der allgemeinen Formel XIII

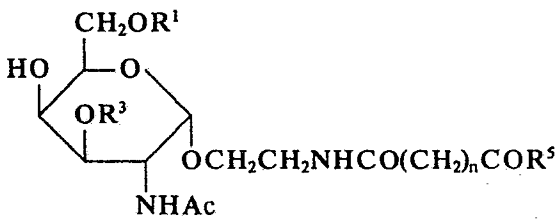

(XIII)

worin $R^1$ eine Acetyl- oder Benzoyl-Gruppe und $R^3$ eine Acetyl-, Benzoyl- oder Benzyl-Gruppe bedeuten, gewinnt,

j) das Produkt der Formel XIII von Stufe i) in an sich bekannter Weise wie bei Stufen e) und h) mit dem Halogenid der Formel X zu einem Disaccharid-Derivat umsetzt und nach entsprechender Entfernung der Schutzgruppen eine Verbindung der Formel VI erhält und

k) in den Produkten der Formel IV bis VII in an sich bekannter Weise die terminale Carbonsäureester-Gruppierung des Brückenbildungsarmes zu $R^5$ = OH, $-NH-NH_2$ oder $N_3$ umsetzt.

Die Produkte von Stufe k) können in an sich bekannter Weise an lösliche Träger über deren terminale Aminogruppe unter Bildung einer Säureamid-Gruppierung gebunden werden.

Solche Träger sind zum Beispiel Peptide und Proteine, bevorzugt humanes oder Rinder-Serum-Albumin, weiße oder rote Blutkörperchen, aminierte Kunststoffe, aminiertes Kieselgel, Aminogruppen tragende Kunststoffe wie aminierte Polyacryl, aminierte Polysaccharide wie Sephadex oder Lipide mit primären Aminofunktionen. Die an Träger gebundenen Verbindungen der Formel I stellen die gewünschten synthetischen T-Antigene dar.

Schema I

Verbindung 1

$HOCH_2CH_2NHCO(CH_2)_4COOCH_3$

Verbindung 2

$HOCH_2CH_2NHCO(CH_2)_7COOCH_3$

Verbindung 3

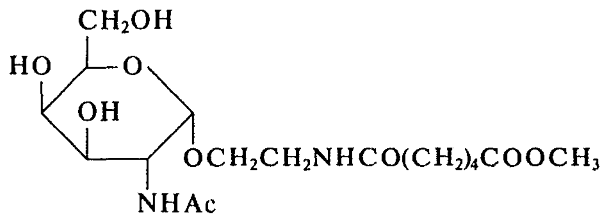

Verbindung 4

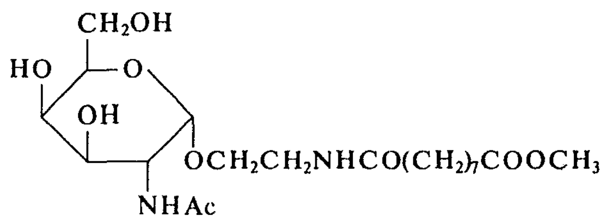

Verbindung 5

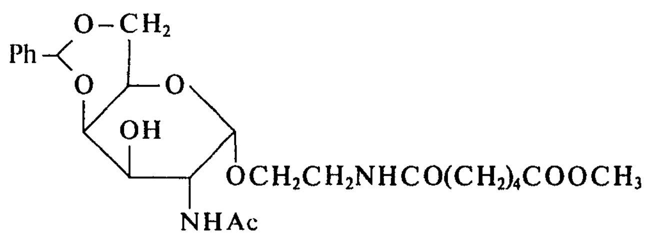

Verbindung 6

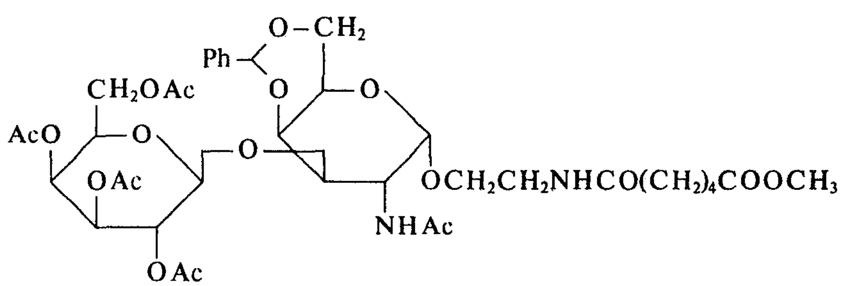

Verbindung 7

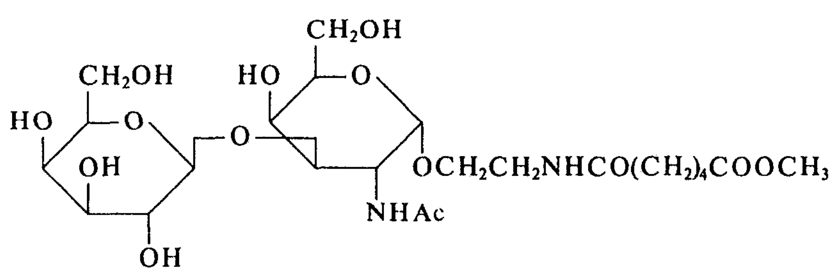

Verbindung 8

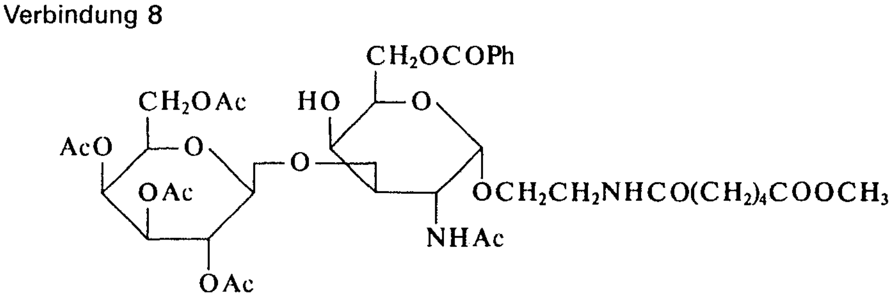

Verbindung 9

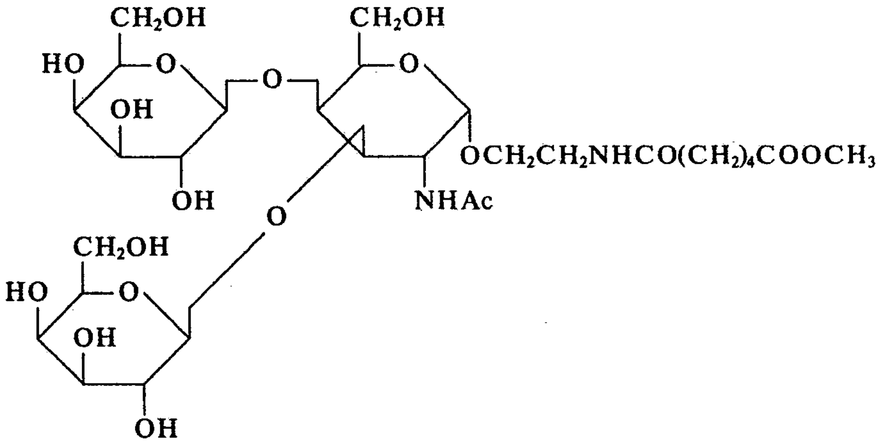

Verbindung 10

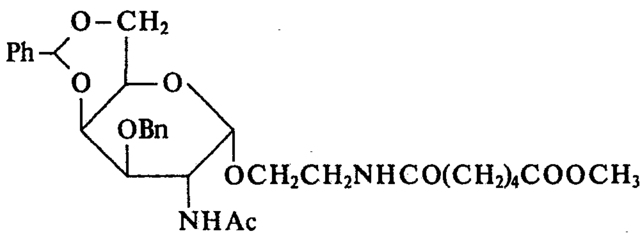

Bn = $PhCH_2-$

Verbindung 11

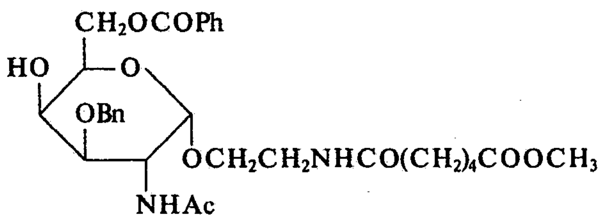

Bn = $PhCH_2-$

Verbindung 12

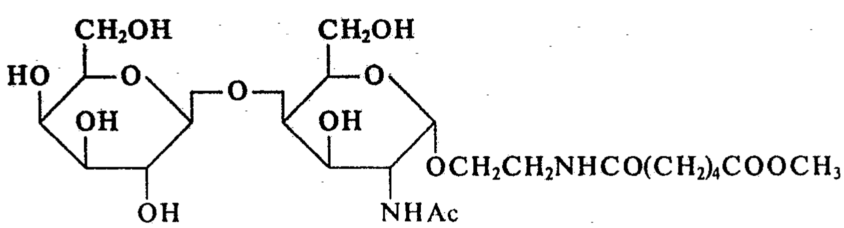

Beispiel 1

Herstellung des N-(5-Methoxycarbonylvaleryl-)-2-aminoethanols (Verbindung 1)

Die Strukturformeln der wesentlichen in den Beispielen genannten Verbindungen sind im Schema I besonders aufgeführt

Methode a

1,6 g (10 mMol) Adipinsäuremonomethylester, gelöst in 20 ml trockenem Dimethylformamid (DMF) werden in Gegenwart von 0,5 g trockenem Molekularsieb mit einer Porenweite 3 Å mit 0,6 ml

(10 mMol) 2-Aminoethanol versetzt. Nach der Zugabe von 2,06 g (10 mMol) Dicyclohexylcarbodiimid wird die Suspension 16 Stunden bei Raumtemperatur gerührt. Es wird abfiltriert, mit Chloroform gewaschen und im Vakuum eingeengt. Die Reinigung des Rohproduktes erfolgt säulenchromatographisch (50 g Kieselgel, Elutionsmittel $CH_2Cl_2/CH_3OH$ 10:1).
Ausbeute 1,62 g (80%).

Methode b

2,02 g (10 mMol) Adipinsäuremonomethylester, gelöst in 20 ml trockenem Tetrahydrofuran werden bei 0°C mit 1,19 g (11 mMol) Chlorameisensäureethylester versetzt. Unter Rühren werden 1,21 g (12 mMol) Triethylamin zugetropft. Nach einer Stunde Rühren bei Raumtemperatur wird abfiltriert und das Filtrat im Vakuum eingeengt. Der gewonnene Sirup wird in trockenem Tetrahydrofuran (THF) gelöst und mit 1,8 ml (30 mMol) 2-Aminoethanol bei 0°C versetzt. Nach 16 Stunden Rühren bei Raumtemperatur wird im Vakuum eingeengt und der Rest mit Chloroform versetzt. Es wird mit Wasser gewaschen, über Natriumsulfat getrocknet und im Vakuum eingeengt. Das Rohprodukt kristallisiert in der Kälte aus Diethylether.
Ausbeute 1,22 g (60%).
Schmp. 16°C; $^1$H-NMR (270 MHz; $CDCl_3$).
M.G. = 203,24.

Beispiel 2

Herstellung des N-(8-Methoxycarbonylcapryl-)-2-Aminoethanols (Verbindung 2)

Die Verbindung 2 wird in gleicher Weise, wie im Beispiel 1 Methoden a und b, beschrieben, aus Azelainsäuremonoethylester und 2-Aminoethanol dargestellt.
Schmp. = 56–57°C; $^1$H-NMR (100 MHz; $CDCl_3$).
M.G. = 245,32.

Beispiel 3

N-(5-Methoxycarbonylvaleryl-)-2-aminoethyl-2-acetamido-2-desoxy-alpha-D-galactopyranosid (Verbindung 3)

2,03 g (10 mMol) 1 werden in 80 ml absolutem Nitromethan gelöst und 3,4 g (13 mMol) Quecksilber(II)cyanid und 0,34 g (0,94 mMol) Quecksilber(II)bromid unter Rühren in die Reaktionsmischung gegeben. Bei Raumtemperatur werden 3,5 g (10 mMol) 3,4,6-Tri-O-acetyl-2-azido-2-desoxy-$\beta$-D-galactopyranosylchlorid, gelöst in 30 ml absolutem Nitromethan, innerhalb von 1 Stunde unter $N_2$-Atmosphäre zugetropft. Die Mischung wird 24 Stunden bei Raumtemperatur gerührt. Nach Zugabe von 200 ml Chloroform wird zweimal mit gesättigter $NaHCO_3$-Lösung und zweimal mit wäßriger gesättigter NaCl-Lösung gewaschen. Die wäßrige Phasen werden zweimal mit $CH_2Cl_2$ extrahiert. Die vereinigten organischen Phasen werden mit $Na_2SO_4$ getrocknet, filtriert und im Vakuum eingedampft. Das Rohprodukt wird in 40 ml Eisessig/Acetanhydrid 1 : 1 gelöst, und 5 g Zinkpulver werden unter Rühren zugegeben. Nach 20 Minuten werden die Feststoffe abfiltriert und das Filtrat im Vakuum eingeengt. Nach mehrmaligem Abdampfen im Vakuum unter jedesmaliger Zugabe von Toluol wird der Sirup 2 Stunden im Hochvakuum getrocknet. Das Rohprodukt wird in 50 ml absolutem Methanol gelöst und mit methanolischer 1N-Natriummethylat-Lösung versetzt. Nach 24 Stunden wird mit Dowex 50 WX-8 ($H^{\oplus}$) neutralisiert. Die säulenchromatische Reinigung erfolgt auf 80 g Kieselgel mit Chloroform/Methanol 4:1.
Ausbeute 1,62 g (40%).
Das Produkt kristallisiert aus Methanol/Ether.
Schmp. = 206°C, $[alpha]_D^{20}$ = +104° (c = 1, $H_2O$)
$^1$H-NMR (270 MHz, DMSO-$d_6$): delta = 4,57 d (1-H $J_{1,2}$ = 3,6 Hz);
4,06 ddd (2-H, $J_{2,3}$ = 11,0 Hz, $J_{2,NH}$ = 8,9 Hz); 1,84 s (AcN); 3,56 s ($OCH_3$).

Beispiel 4

N-(8-Methoxycarbonylcapryl-)-2-aminoethyl-alpha-acetamido-2-desoxy-alpha-D-galactopyranosid (Verbindung 4)

Die Verbindung 4 wird in gleicher Weise wie in Beispiel 3 aus 3,4,6-Tri-O-acetyl-2-azido-2-desoxy-$\beta$-D-galactopyranosylchlorid und Verbindung 2 dargestellt.
Schmp. = 234°C; $[alpha]_D^{20}$ = +89°C (c = 1, $H_2O$); $^1$H-NMR (270 MHz, DMSO-$d_6$).

## Beispiel 5

N-(5-Methoxycarbonylvaleryl-)-2-aminoethyl-2-acetamido-4,6-O-benzyliden-2-desoxy-alpha-D-galactopyranosid (Verbindung 5)

1,75 g (4,3 mMol) Verbindung 3 werden mit 20 ml frisch destilliertem Benzaldehyd und 1,5 g pulverisiertem Zink (II)chlorid (vorher zur Entwässerung geschmolzen) versetzt. Der Ansatz wird 24 Stunden geschüttelt und dann in 60 ml eiskalte gesättigte wäßrige $NaHCO_3$-Lösung gegossen. Nach der Extraktion mit Chloroform wird die organische Phase mit Wasser gewaschen, mit $Na_2SO_4$ getrocknet und im Vakuum eingeengt. Das Rohprodukt kristallisiert aus heißem Ethanol.
Ausbeute 1,7 g (80%).
Schmp. 221°C; $[alpha]_D^{20} = +110°$ (c = 0,5 in $CHCl_3/CH_3OH$ (1 : 1)
$^1$H-NMR (270 MHz, $DMSO^D$-$d_6$): delta = 5,56 s (C<u>H</u>Ph); 4,69 d (1-H, $J_{1,2}$ = 3,4 Hz); 4,07 ddd (2-H, $J_{2,3}$ = 11,1 Hz, $J_{2,NH}$ = 8,5 Hz); 3,79 m (3-H, $J_{3,4}$ = 3,4 Hz); 3,55 s ($OCH_3$) 1,25 s (NAc).

## Beispiel 6

N-(5-Methoxycarbonyl-)-2-aminoethyl-2-acetamido-3-O-(2,3,4,6-tetra-O-acetyl-β-D-galactopyranosyl)-4,6-O-benzyliden-2-desoxy-alpha-D-galactopyranosid (Verbindung 6)

0,49 g (1 mMol) 5 werden in 16 ml absolutem Nitromethan/Benzol 3 : 1 gelöst und 0,51 g (2 mMol) Quecksilber (II) cyanid und 36 mg (0,1 mMol) Quecksilber (II) bromid unter Rühren zugegeben. Bei 60°C werden 0,82 g (2 mMol) 2,3,4,6-Tetra-O-acetyl-alpha-D-galactopyranosylbromid, gelöst in 8 ml Benzol, innerhalb von 4 Stunden unter $N_2$-Atmosphäre zugetropft. Nach 16 Stunden wird das Reaktionsgemisch abfiltriert, mit $CH_2Cl_2$ nachgewaschen. Das Filtrat wird zweimal mit wäßriger gesättigter $NaHCO_3$-Lösung und zweimal mit wäßriger gesättigter NaCl-Lösung gewaschen. Die wäßrige Phasen werden zweimal mit $CH_2Cl_2$ extrahiert. Die vereinigten organischen Phasen werden mit $Na_2SO_4$ getrocknet, filtriert und im Vakuum eingedampft. Die säulenchromatographische Reinigung erfolgt auf 80 g Kieselgel mit $CH_2Cl_2$/Methanol 12 : 1.
Ausbeute 0,71 g (86%) amorphes Produkt;
$[alpha]_D^{20} = +81°$ (c = 1, $CHCl_3$).
$^1$H-NMR (270 MHz, $CDCl_3$) delta: 4,76 d (1'-H $J_{1',2'}$ = 7,8 Hz), 5,19 dd (2'-H $J_{2',3'}$ = 10,4 Hz), 2,14 m (4-OAcn · NAc).

## Beispiel 7

n-(5-Methoxycarbonylvaleryl-)-2-aminomethyl-2-acetamido-2-desoxy-3-O-(β-D-galactopyranosyl)-alpha-D-galactopyranosid (Verbindung 7)

650 mg (0,78 mMol) 6 werden in 20 ml 60%iger Essigsäure 1/2 Stunde auf 80°C erwärmt. Anschließend wird die Lösung im Vakuum eingedampft. Der in 50 ml gelöste Sirup wird mit Wasser, gesättigter $NaHCO_3$-Lösung und Wasser gewaschen. Trocknen und Eindampfen im Vakuum liefert 585 mg Sirup. Das Rohprodukt wird in 15 ml absolutem Methanol gelöst, 2 ml 0,2 N $NaOCH_3$ zugegeben und 24 Stunden bei Raumtemperatur stehengelassen. Es wird mit Amberlite IR 120 $H^+$ neutralisiert, filtriert und im Vakuum eingedampft. Die zurückbleibende kristalline Masse wird aus Methanol umkristallisiert.
Ausbeute 360 mg (80%);
Schmp. = 191°C, $[alpha]_D^{20} = +76,5°$ (c = 1, $H_2O$)
$^1$H-NMR (270 MHz, DMSO-$d_6$): delta = 4,57 d (1-H $J_{1,2}$ = 3,5 Hz); 4,24 ddd (2-H $J_{2,3}$ = 12,0 Hz; $J_{2,NH}$ = 8,4 Hz); 4,3 d (1'-H $J_{1',2'}$ = 7,3 Hz); 1,84 s (N<u>Ac</u>); 3,57 s ($OCH_3$).

## Beispiel 8

N-(5-Methoxycarbonylvaleryl-)-2-aminoethyl-2-acetamido-3-O-(2,3,4,6-tetra-O-acetyl-β-D-galactopyranosyl)-6-O-benzoyl-2-desoxy-alpha-D-galactopyranosid (Verbindung 8)

650 mg (0,78 mMol) 6 werden in 60 ml 60%iger Essigsäure 1/2 Stunde auf 80°C erwärmt. Anschließend wird die Lösung in Vakuum eingedampft, der resultierende Sirup mehrmals unter jeweiliger Zugabe von Toluol im Vakuum abgedampft und in Hochvakuum getrocknet. Das Produkt $[alpha]_D^{20} = +51°$ (c = 1, $CHCl_3$) wird in 30 ml $CH_2Cl_2$ und 15 ml Pyridin gelöst. Nach der Zugabe von 160 mg (1,3 mMol) Benzoylcyanid wird 7 Stunden bei Raumtemperatur gerührt. Dann werden 15 ml Methanol zugegeben und zum Sirup eingeengt. Der Sirup wird säulenchromatisch gereinigt (60 g Kieselgel;

Elutionsmittel $CH_2Cl_2$/Methanol 14:1).
Ausbeute: 550 mg (83%).
Schmp.: 171°C; $[alpha]_D^{21}$ = +42° (c = 1 $CHCl_3$/Methanol (1:1))
$^1$H-NMR (270 MHz, $CDCl_3$) delta = 7,45–7,58 m (Ph) 4,88 d (1-H $J_{1,2}$ = 3,4 Hz); 4,68 d (1'-H $J_{1',2'}$ = 7,9 Hz) 5,22 dd (2'-H $J_{2',3'}$ = 10,4 Hz); 5,01 dd (3'-H $J_{3',4'}$ = 3,6 Hz); 1,99 s; 2,02 s; 2,04 s; 2,10 s; 2,17 s (4 OAc + NAc).

## Beispiel 9

N-(5-Methoxycarbonylvaleryl-)-2-aminoethyl-2-acetamido-4-O-(β-D-galactopyranosyl)-3-O-(β-D-galactopyranosyl)-alpha-D-galactopyranosid (Verbindung 9)

340 mg (0,4 mMol) 8 werden in 12 ml absolutem Nitromethan/Benzol 2:1 gelöst und 260 mg (1 mMol) Quecksilber (II) cyanid und 36 mg (0,01 mMol) Quecksilber (II) bromid bei 60°C zugegeben. Unter Rühren werden 320 mg (0,8 mMol) 2,3,4,6-Tetra-O-acetyl-alpha-D-galactopyranosylbromid in 4 ml Benzyl zugetropft. Die weitere Aufarbeitung erfolgt wie im Beispiel 6. Das Produkt kristallisiert aus Ether/Hexan).
Ausbeute: 360 mg (76%);
Schmp.: 149°C; $[alpha]_D^{20}$ = +22° (c = 1 $CHCl_3$)
$^1$H-NMR (270 MHz, $CDCl_3$): delta = 4,75 d (1-H $J_{1,2}$ = 3,7 Hz); 4,69 d (1'-H $J_{1',2'}$ = 7,8 Hz); 5,05 d (1''-H $J_{1'',3''}$ = 7,8 Hz).
Das peracetylierte Produkt wird in 20 ml Methanol gelöst, und 2 ml 0,2 N $NaOCH_3$ werden zugegeben. Nach 24 Stunden wird mit Amberlite IR 120 $H^+$ neutralisiert, filtriert und in Vakuum eingedampft. Das Produkt wurde mehrere Stunden im Hochvakuum über $P_2O_5$ getrocknet.
Ausbeute: 205 mg (92%) amorph;
$[alpha]_D^{20}$ = +15,6° (c = 1 $H_2O$).

## Beispiel 10

N-(5-Methoxycarbonylvaleryl)-2-aminomethyl-2-acetamido-3-O-benzyl-4,6-O-benzyliden-2-desoxy-alpha-D-galactopyranosid (Verbindung 10)

495 mg (1 mMol) 5 werden in 25 ml absolutem DMF gelöst. Nach Zugabe von 1,38 g (9 mMol) BaO und 315 mg (1 mMol) $Ba(OH)_2 \cdot 8\,H_2O$ werden 0,6 ml (5 mMol) Benzylbromid zugegeben. Nach 7 Stunden Rühren unter Feuchtigkeitsausschluß werden 3 ml Methanol zugegeben. Dann wird filtriert, mit viel Chloroform nachgewaschen und im Vakuum eingeengt. Das Produkt wird säulenchromatographisch gereinigt (40 g Kieselgel, Elutionsmittel $CH_2Cl_2$/Methanol 12:1).
Ausbeute: 456 mg (78%);
Schmp.: 209°C; $[alpha]_D^{20}$ = +125° (c = 1 $CHCl_3/CH_3OH$ 1:1)
$^1$H-NMR (270 MHz, DMSO-$d_6$): delta = 7,35 m (Ph); 5,62 s (Ph<u>CH</u>); 4,58 dd (Ph$C\underline{H_2}$); 4,75 d (1-H $J_{1,2}$ = 3,2 Hz); 4,29 dd (2-H $J_{2,3}$ = 11,6 Hz); 3,82 dd (3-H $J_{3,4}$ = 3,0 Hz).

## Beispiel 11

N-(5-Methoxycarbonylvaleryl)-2-aminoethyl-2-acetamido-6-O-benzoyl-3-O-benzyl-2-desoxy-alpha-D-galactopyranosid (Verbindung 11)

450 mg (0,77 mMol) 10 werden in 20 ml 60%iger Essigsäure 1 Stunde bei 80°C erwärmt. Anschließend wird die Lösung in Vakuum eingedampft, der resultierende Sirup mehrmals mit Toluol versetzt und das Toluol jeweils im Vakuum abdestilliert und der Sirup im Hochvakuum getrocknet. Das Rohprodukt (378 mg) wird ohne weitere Reinigung wie im Beispiel 8 weiterverarbeitet.
Der resultierende Sirup kristallisiert aus Ethanol.
Ausbeute: 381 mg (83%);
Schmp.: = 211°C; $[alpha]_D$ = +72° (c = 1 $CHCl_3/CH_3OH$ 1:1)
$^1$H-NMR (270 MHz, DMSO-$d_6$) delta: 4,62 d (1-H $J_{1,2}$ = 3,8 Hz); 4,34 ddd (2-H $J_{1,2}$ Hz, $J_{2,NH}$ = 8,6 Hz); 3,65 dd (3-H $J_{3,4}$ = 2,8 Hz).

## Beispiel 12

N-(5-Methoxycarbonylvaleryl)-2-aminomethyl-2-acetamido-2-desoxy-4-O-(β-D-galactopyranosyl)-alpha-D-galactopyranosid (Verbindung 12)

300 mg (0,5 mMol) 11 werden in 18 ml absolutem Nitromethan/Benzol 2:1 gelöst, 256 mg (1 mMol) Quecksilber (II) cyanid und 36 mg (0,1 mMol) Quecksilber (II) bromid bei 60°C zugegeben.

Unter Rühren werden 411 mg (1 mMol) 2,3,4,6-Tetra-O-acetyl-alpha-D-galactopyranosylbromid in 4 ml Benzol zugetropft. Die weitere Aufarbeitung erfolgt wie in Beispiel 6.

Das Rohprodukt (340 mg) wird in 15 ml Eisessig gelöst und in Gegenwart von 200 mg Palladium/Kohle (10%) 5 Stunden bei Raumtemperatur hydriert. Es wird mit 20 ml Chloroform verdünnt, filtriert, nachgewaschen und im Vakuum eingeengt. Der Rückstand wird in 30 ml $CHCl_3$ aufgenommen, mit gesättigter $NaHCO_3$-Lösung und Wasser gewaschen. Das Produkt wird säulenchromatographisch gereinigt (60 g Kieselgel; Elutionsmittel $CH_2Cl_2$/Methanol 14:1).
Ausbeute 262 g (86%);
Schmp.: 151°C; $[\text{alpha}]_D^{20} = +44°$ (c = 1 $CHCl_3/CH_3OH$ 1:1)
$^1$H-NMR (270 MHz, DMSO-$d_6$) delta = 4,61 d (1-H $J_{1,2} = 3{,}6$ Hz); 4,90 d (1'-H $J_{1',2'} = 8{,}6$ Hz); 4,88 dd (2'-H $J_{2',3'} = 8{,}6$ Hz); 1,84–2,11 s (5 OAc + NAc).

Das Produkt wird in 30 ml absolutem Methanol gelöst und 2 ml 0,2 N $NaOCH_3$ werden zugegeben. Nach 24 Stunden Stehen wird mit Amberlite IR 120 $H^+$ neutralisiert, filtriert und im Vakuum eingedampft. Die zurückbleibende kristalline Masse wird aus Methanol/Ethanol umkristallisiert.
Ausbeute: 166 mg (87%); $[\text{alpha}]_D^{20} = +23°$ (c = 1 $H_2O$).

**Patentansprüche für die Vertragsstaaten: BE, CH, DE, FR, GB, IT, LI, LU, NL, SE**

1. Eine Verbindung der allgemeinen Formel I

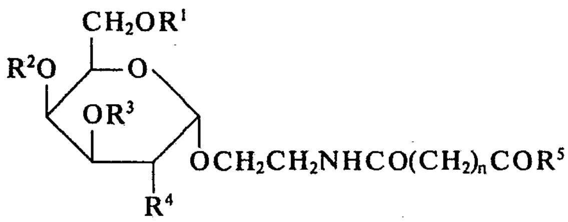

(I)

worin

| | |
|---|---|
| $R^1$ | ein Wasserstoffatom oder eine Acyl- oder Benzyl-Schutzgruppe, |
| $R^1$ und $R^2$ | zusammen eine Alkyliden- oder Benzyliden-Schutzgruppe, |
| $R^2$ und $R^3$ | Wasserstoffatome oder Acyl- oder Benzyl-Schutzgruppen oder β-D-Galactopyranosyl-Reste oder deren 2,3,4,6-Tetra-O-acetylierte Derivate, |
| $R^4$ | eine Azido-, Amino- oder Acetamido-Gruppe, |
| $R^5$ | –O-Alkyl, –O-Aryl, –O-Benzyl, $-NHNH_2$, $-N_3$ oder –OH und |
| n | eine ganze Zahl von 1–10 |

bedeuten.

2. Eine Verbindung nach Anspruch 1, dadurch gekennzeichnet, daß

| | |
|---|---|
| $R^1$ | ein Wasserstoffatom oder eine Acetyl- oder Benzoyl-Schutzgruppe, |
| $R^1$ und $R^2$ | zusammen eine Isopropyliden- oder Benzyliden-Schutzgruppe, |
| $R^2$ und $R^3$ | Wasserstoffatome, Acetyl-, Benzoyl- oder Benzyl-Schutzgruppen, β-D-Galactopyranosyl-Reste oder deren 2,3,4,6-Tetra-O-acetylierte Derivate, |
| $R^4$ | eine Azido-, Amino- oder Acetamido-Gruppe, |
| $R^5$ | $-OCH_3$, $-NHNH_2$, $-N_3$ oder –OH und |
| n | eine ganze Zahl von 4 bis 7 |

bedeuten.

3. Eine Verbindung nach Anspruch 1, dadurch gekennzeichnet, daß

| | |
|---|---|
| $R^1$, $R^2$ und $R^3$ | Wasserstoffatome, |
| $R^4$ | eine Acetamidogruppe, |
| $R^5$ | $-OCH_3$ und |
| n | 4 oder 7 sind. |

4. Eine Verbindung nach Anspruch 1, dadurch gekennzeichnet, daß

| | |
|---|---|
| $R^1$ | ein Wasserstoffatom, |
| $R^2$ und $R^3$ | β-D-Galactopyranosyl-Reste, |
| $R^4$ | eine Acetamidogruppe, |
| $R^5$ | $-OCH_3$ und |
| n | 4 sind. |

5. Eine Verbindung nach Anspruch 1, dadurch gekennzeichnet, daß

| | |
|---|---|
| $R^1$ und $R^3$ | Wasserstoffatome, |
| $R^2$ | ein β-D-Galactopyranosyl-Rest, |
| $R^4$ | eine Acetamidogruppe, |
| $R^5$ | $-OCH_3$ und |
| n | 4 sind. |

6. Eine Verbindung nach Anspruch 1, dadurch gekennzeichnet, daß

| | |
|---|---|
| $R^1$ und $R^2$ | Wasserstoffatome, |
| $R^3$ | ein β-D-Galactopyranosyl-Rest |
| $R^4$ | eine Acetamidogruppe |
| $R^5$ | $-OCH_3$ und |
| n | = 4 sind. |

7. Verfahren zur Herstellung einer Verbindung nach Anspruch 1, dadurch gekennzeichnet, daß man eine Verbindung der Formel II

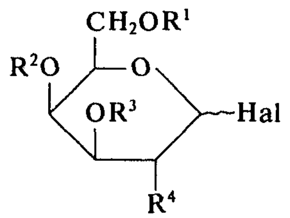

(II)

in der

| | |
|---|---|
| $R^1$, $R^2$ und $R^3$ | Acylgruppen, |
| $R^4$ | eine Azidogruppe und |
| Hal | Chlor, Brom oder Jod bedeuten, |

mit einer Verbindung der Formel III

$$HOCH_2CH_2NHCO(CH_2)_nCOR^5 \quad (III)$$

worin $R^5$ —O-Alkyl oder —O-Benzyl und eine ganze Zahl von n 1 bis 10 bedeuten, zu einer Verbindung der Formel I, in der $R^1$, $R^2$, $R^3$ und $R^4$ die zu Formel II und $R^5$ und n die zu der Formel III angegebene Bedeutung haben, umsetzt.

8. Verwendung einer Verbindung nach Anspruch 1 in aktivierter Form, wobei $R^5$ $-N_3$ oder —OH ist, zur Bindung an einen Träger.

9. Verwendung einer an einen Träger gebundenen Verbindung nach Anspruch 1 als Antigen in einem Tier zur Gewinnung von Antikörpern oder in einem Diagnostikum oder Immunoadsorbens.

**Patentansprüche für den Vertragsstaat AT**

1. Ein Verfahren zur Herstellung einer Verbindung der allgemeinen Formel I

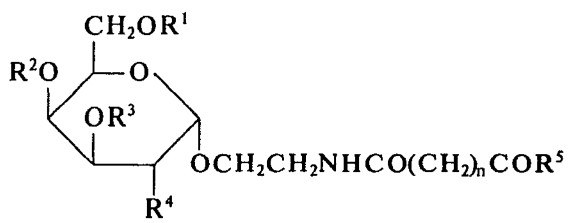

(I)

worin

| | |
|---|---|
| $R^1$ | ein Wasserstoffatom, |
| $R^2$ und/oder $R^3$ | Wasserstoffatome oder β-D-Galactopyranosyl-, |
| $R^4$ | eine Acetamidogruppe, |
| $R^5$ | —O-Alkyl, —O-Aryl oder —O-Benzyl und |
| n | eine ganze Zahl von 1 bis 10 |

bedeuten, dadurch gekennzeichnet, daß man eine Verbindung der Formel I worin

| | |
|---|---|
| $R^1$ | ein Wasserstoffatom, |
| $R^2$ und/oder $R^3$ | 2,3,4,6-Tetra-O-acetyl-$\beta$-D-Galactopyranosyl und |
| $R^3$ oder $R^2$ | ein Wasserstoffatom, |
| $R^4$ | eine Acetamidogruppe, |
| $R^5$ | —O-Alkyl, —O-Aryl oder —O-Benzyl und |
| n | eine ganze Zahl von 1 bis 10 |

bedeuten, mit einer Base behandelt.

2. Verwendung einer Verbindung der Formel I gebunden an einen Träger.

3. Verwendung einer Verbindung nach Anspruch 2 als Antigen in einem Tier zur Gewinnung von Antikörpern oder in einem Diagnostikum oder Immunoadsorbens.

**Claims for the Contracting States: BE, CH, DE, FR, GB, IT, LI, LU, NL, SE**

1. A compound of the general formula I

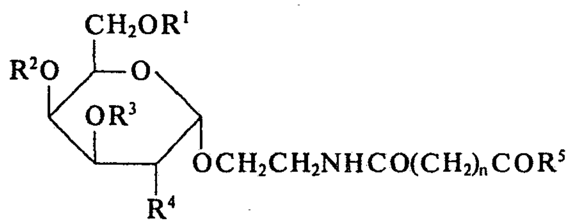

(I)

wherein

| | |
|---|---|
| $R^1$ | denotes a hydrogen atom or an acyl or benzyl protective group, |
| $R^1$ and $R^2$ | together denote an alkylidene or benzylidene protective group, |
| $R^2$ and $R^3$ | denote hydrogen atoms or acyl or benzyl protective groups or $\beta$-D-galactopyranosyl radical or 2,3,4,6-tetra-O-acetylated derivatives thereof, |
| $R^4$ | denotes an azido, amino or acetamido group, |
| $R^5$ | denotes —O-alkyl, —O-aryl, —O-benzyl, $-NHNH_2$, $-N_3$ or —OH and |
| n | denotes a whole number from 1 to 10. |

2. A compound as claimed in claim 1, wherein

| | |
|---|---|
| $R^1$ | denotes a hydrogen atom or an acetyl or benzoyl protective group, |
| $R^1$ and $R^2$ | together denote an isopropylidene or benzylidene protective group, |
| $R^2$ and $R^3$ | denote hydrogen atoms, acetyl, benzoyl or benzyl protective groups, $\beta$-D-galactopyranosyl residue or 2,3,4,6-tetra-O-acetylated derivatives thereof, |
| $R^4$ | denotes an azido, amino or acetamido group, |
| $R^5$ | denotes $-OCH_3$, $-NHNH_2$, $-N_3$ or —OH and |
| n | denotes a whole number from 4 to 7. |

3. A compound as claimed in claim 1, wherein

| | |
|---|---|
| $R^1$, $R^2$ and $R^3$ | are hydrogen atoms, |
| $R^4$ | is an acetamido group, |
| $R^5$ | is $-OCH_3$ and |
| n | is 4 or 7. |

4. A compound as claimed in claim 1, wherein

| | |
|---|---|
| $R^1$ | is a hydrogen atom, |
| $R^2$ and $R^3$ | are $\beta$-D-galactopyranosyl residue, |
| $R^4$ | is an acetamido group, |
| $R^5$ | is $-OCH_3$ and |
| n | is 4. |

5. A compound as claimed in claim 1, wherein

$R^1$ and $R^3$ are hydrogen atoms,<br>
$R^2$ is a $\beta$-D-galactopyranosyl residue,<br>
$R^4$ is an acetamido group,<br>
$R^5$ is $-OCH_3$ and<br>
n is 4.

6. A compound as claimed in claim 1, wherein

$R^1$ and $R^2$ are hydrogen atoms,<br>
$R^3$ is a $\beta$-D-galactopyranosyl residue,<br>
$R^4$ is an acetamido group,<br>
$R^5$ is $-OCH_3$ and<br>
n is 4.

7. A process for the preparation of a compound as claimed in claim 1, which comprises reacting a compound of the formula II

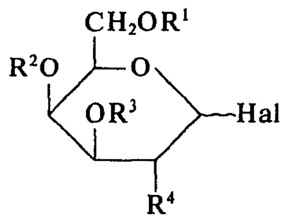

(II)

in which

$R^1$, $R^2$ and $R^3$ denote acyl groups,<br>
$R^4$ denotes an azido group and<br>
Hal denotes chlorine, bromine or iodine,

with a compound of the formula III

$$HOCH_2CH_2NHCO(CH_2)_nCOR^5 \qquad \text{(III)}$$

wherein $R^5$ denotes $-O$-alkyl or $-O$-benzyl and n denotes a whole number from 1 to 10 give a compound of the formula I in which $R^1$, $R^2$, $R^3$ and $R^4$ have the meaning indicated for formula II and $R^5$ and n have the meaning indicated for formula III.

8. Use of a compound as claimed in claim 1 in an activated form, $R^5$ being $-N_3$ or $-OH$, for attachment to a carrier.

9. Use of a compound as claimed in claim 1, attached to a carrier, as an antigen in an animal for producing antibodies or in a diagnosticum or immunoadsorbent.

**Claims for the Contracting State: AT**

1. Process for the preparation of a compound of the general formula I

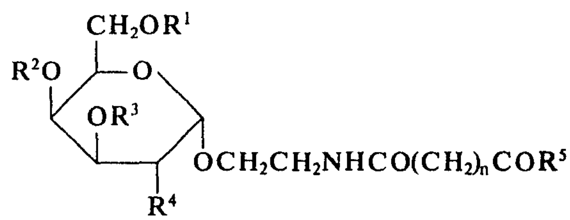

(I)

wherein

$R^1$ is a hydrogen atom,<br>
$R^2$ and/or $R^3$ denote hydrogen atoms of the $\beta$-D-galactopyraosyl radical,<br>
$R^4$ is acetamido group,<br>
$R^5$ denotes $-O$-alkyl, $-O$-aryl or $-O$-benzyl and<br>
n denotes a whole number from 1 to 10,

which comprises reacting a compound of the formula I, wherein

| | |
|---|---|
| $R^1$ | is a hydrogen atom, |
| $R^2$ and/or $R^3$ | denote 2,3,4,6-tetra-O-acetyl-$\beta$-D-galactopyranosyl and |
| $R^3$ or $R^2$ | a hydrogen atom, |
| $R^4$ | is acetamido group, |
| $R^5$ | denotes $-$O-alkyl, $-$O-aryl or $-$O-benzyl and |
| n | denotes a whole number from 1 to 10, |

with a base.

2. Use of a compound of general formula I in claim 1, attached to a carrier.

3. Use of a compound as claimed in claim 2, attached to a carrier, as an antigen in an animal for producing antibodies or in a diagnosticum or immunoadsorbent.

**Revendications pour les Etats contractants: BE, CH, DE, FR, GB, IT, LI, LU, NL, SE**

1. Composés de formule générale I

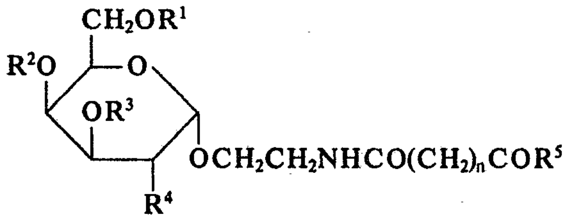

(I)

dans laquelle

| | |
|---|---|
| $R^1$ | représente un atome d'hydrogène ou un groupe protecteur acyle ou benzyle, |
| $R^1$ et $R^2$ | représentent ensemble un groupe protecteur alcoylidène ou benzylidène, |
| $R^2$ et $R^3$ | représentent des atomes d'hydrogène ou des groupes protecteurs acyle ou benzyle ou des radicaux $\beta$-D-galactopyranosyle ou leurs dérivés 2,3,4,6-tétra-O-acétylés, |
| $R^4$ | représente un groupe azido, amino ou acétamido, |
| $R^5$ | représente un groupe $-$O-alcoyle, $-$O-aryle, $-$O-benzyle, $-NHNH_2$, $-N_3$ ou $-$OH, et |
| n | est un nombre entier de 1 à 10. |

2. Composé selon la revendication 1, caractérisé en ce que

| | |
|---|---|
| $R^1$ | représente un atome d'hydrogène ou un groupe protecteur acétyle ou benzoyle, |
| $R^1$ et $R^2$ | représentent ensemble un groupe protecteur isopropylidène ou benzylidène, |
| $R^2$ et $R^3$ | représentent des atomes d'hydrogène, des groupes protecteurs acétyle, benzoyle ou benzyle, des radicaux $\beta$-D-galactopyranosyle ou leurs dérivés 2,3,4,6-tétra-O-acétylés, |
| $R^4$ | représente un groupe azido, amino ou acétamido, |
| $R^5$ | représente $-OCH_3$, $-NHNH_2$, $-N_3$ ou $-$OH et |
| n | est un nombre entier de 4 à 7. |

3. Composé selon la revendication 1, caractérisé en ce que

| | |
|---|---|
| $R^1$, $R^2$ et $R^3$ | représentent des atomes d'hydrogène, |
| $R^4$ | représente un groupe acétamido, |
| $R^5$ | représente $-OCH_3$ et |
| n | est 4 ou 7. |

4. Composé selon la revendication 1, caractérisé en ce que

| | |
|---|---|
| $R^1$ | représente un atome d'hydrogène, |
| $R^2$ et $R^3$ | représentent des radicaux $\beta$-D-galactopyranosyle, |
| $R^4$ | représente un groupe acétamido, |
| $R^5$ | représente $-OCH_3$ et |
| n | est 4. |

5. Composé selon la revendication 1, caractérisé en ce que

| | |
|---|---|
| $R^1$ et $R^3$ | représentent des atomes d'hydrogène, |

$R^2$ représente un radical β-D-galactopyranosyle,
$R^4$ représente un groupe acétamido,
$R^5$ représente $-OCH_3$ et
n est 4.

6. Composé selon la revendication 1, caractérisé en ce que

$R^1$ et $R^2$ représentent des atomes d'hydrogène,
$R^3$ représente un radical β-D-galactopyranosyle,
$R^4$ représente un groupe acétamido,
$R^5$ représente $-OCH_3$ et
n est 4.

7. Procédé pour la préparation d'un composé selon la revendication 1, caractérisé en ce qu'on fait réagir un composé de formule II

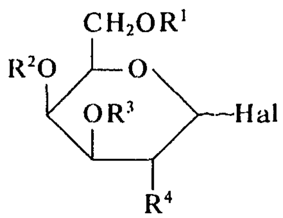

(II)

dans laquelle

$R^1$, $R^2$ et $R^3$ représentent des groupes acyle,
$R^4$ représente un groupe azido et
Hal représente le chlore, le brome ou l'iode,

avec un composé de formule III

$HOCH_2CH_2NHCO(CH_2)_nCOR^5$ (III)

dans laquelle $R^5$ représente un groupe O-alcoyle ou O-benzyle et n est un nombre entier de 1 à 10, pour obtenir un composé de formule I, dans lequel $R^1$, $R^2$, $R^3$ et $R^4$ ont les significations indiquées pour la formule II et $R^5$ et n ont les significations indiquées pour la formule III.

8. Utilisation d'un composé selon la revendication 1 sous une forme active, dans lequel $R^5$ est $-N_3$ ou $-OH$, pour fixation sur un support.

9. Ultilisation d'un composé selon la revendication 1, fixé sur un support, en tant qu'antigène chez un animal pour obtenir des anticorps, ou dans un diagnostic ou en tant qu'immunoadsorbant.

**Revendications pour l'Etatcontractant: AT**

1. Procédé pour la préparation d'un composé de formule générale I

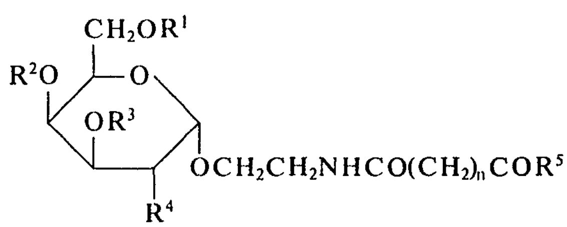

(I)

dans laquelle

$R^1$ représente un atome d'hydrogène,
$R^2$ et/ou $R^3$ représentent des atomes d'hydrogène ou des radicaux β-D-galactopyranosyle,
$R^4$ représente un groupe acétamido,
$R^5$ représente un groupe O-alcoyle, O-aryle ou O-benzyle et
n est un nombre entier de 1 à 10,

caractérisé en ce qu'on traite un composé de formule I, dans lequel

| | |
|---|---|
| $R^1$ | représente un atome d'hydrogène, |
| $R^2$ et/ou $R^3$ | représentent un radical 2,3,4,6-tétra-O-acétyl-$\beta$-D-galactopyranosyle et $R^2$ ou $R^3$ représente un atome d'hydrogène, |
| $R^4$ | représente un groupe acétamido, |
| $R^5$ | représente un groupe O-alcoyle, O-aryle ou O-benzyle et |
| n | est un nombre entier de 1 à 10, |

avec une base.

2. Utilisation d'un composé de formule I fixé sur un support.

3. Utilisation d'un composé selon la revendication 2 en tant qu'antigène chez un animal pour obtenir des anticorps ou dans un diagnostic ou en tant qu'immunoadsorbant.